# EUROPÄISCHE PATENTSCHRIFT

(11) **EP 3 233 182 B1**
(45) Veröffentlichungstag und Bekanntmachung des Hinweises auf die Patenterteilung: **13.06.2018**
(21) Anmeldenummer: 15823317.1
(22) Anmeldetag: 23.10.2015
(51) Int. Cl.: A61N 1/36, A61N 1/04

(54) **EMS-TRAININGSVORRICHTUNG, SOWIE VERFAHREN ZUM SCHUTZ EINER EMS-TRAININGSVORRICHTUNG**
EMS TRAINING DEVICE, AND METHOD FOR PROTECTING AN EMS TRAINING DEVICE
DISPOSITIF D'ENTRAÎNEMENT ESM (ÉLECTROSTIMULATION MUSCULAIRE), ET PROCÉDÉ DE PROTECTION D'UN DISPOSITIF D'ENTRAÎNEMENT ESM

(30) Priorität: 16.12.2014 DE 102014018607
(43) Veröffentlichungstag der Anmeldung: 25.10.2017
(73) Patentinhaber: miha bodytec GmbH, 86368 Gersthofen (DE)
(72) Erfinder: HORTER, Hansjürgen, 72644 Oberboihingen (DE); DECKER, Jürgen, 86494 Emersacker (DE)
(74) Vertreter: Munk, Ludwig Hubert
(86) Internationale Anmeldenummer: PCT/EP2015/025073
(87) Internationale Veröffentlichungsnummer: WO 2016/096151

(56) Entgegenhaltungen:
- EP-A1- 2 024 020
- EP-A2- 0 965 358
- WO-A1-2005/107849
- WO-A2-2014/000736
- DE-U1-202011 109 226

## Beschreibung

Die Erfindung betrifft eine EMS-Trainingsvorrichtung gemäß dem Oberbegriff des Anspruchs 1, sowie ein Verfahren zum Schutz einer solchen EMS-Trainingsvorrichtung gemäß dem Oberbegriff des Anspruchs 15.

Mit Elektromuskelstimulation (EMS), teilweise auch Elektromyostimulation genannt werden Muskeln im lebenden Körper, im Regelfall zu Muskelaufbauzwecken, beispielsweise in Fitness-Studios oder bei Personal-Trainern mit elektrischen Reizen beaufschlagt, um die Muskeln zu kräftigen. Eine derartige EMS-Trainingsvorrichtung ist aus EP 2 024 020 B1 bekannt.

Dabei weisen übliche, am Körper anzulegende EMS-Elektroden häufig Textilstrukturen mit eingearbeiteten Metallfäden, insbesondere Silber- oder Kupfermetallfäden auf, oder Pads wie Polymer-Pads, die mit leitfähigen Partikeln (z.B. Ruß) gefüllt sind. Eine EMS-Elektrode ist beispielsweise der deutschen Patentanmeldung DE 10 2007 046 886 A1 zu entnehmen.

Derartige EMS-Elektroden werden meistens vor dem Training genässt oder über einem genässten Unterbekleidungsstück, z.B. T-Shirt getragen, wozu sie in der Regel an einem am Körper anlegbaren Elektrodenträger angebracht sind. Während frühere Elektrodenträger häufig aus Riemen- und Lederbändern bestanden, an denen die Elektroden angebracht waren, geht die Entwicklung immer mehr hin zu EMS-Kleidungsstücken, insbesondere textilen Kleidungsstücken, die die Elektroden tragen und selbst von der trainierenden Person eben wie ein Kleidungsstück getragen werden können, also beispielsweise als Weste, Hose, Strumpf, Armband oder dergleichen. Um die Akzeptanz der Elektromuskelstimulation (EMS) dabei weiter zu erhöhen, wird versucht, die Elektroden und die an die Elektroden angeschlossenen Leiterbahnen immer besser in das EMS-Kleidungsstück zu integrieren, um so die sichtbaren Kabel zu eliminieren oder zumindest auf die von dem EMS-Kleidungsstück zu einer externen Steuereinheit führenden Kabel zu verkürzen, sowie die Elektroden nicht nur weitestgehend unsichtbar zu machen, sondern auch den Tragekomfort des EMS-Kleidungsstücks allgemein zu erhöhen, in dem die EMS-Elektroden möglichst flexibel und dehnbar, häufig aus textilen Materialien, gebildet werden. Ein Beispiel für ein solches EMS-Kleidungsstück ist dem deutschen Patent DE 10 2009 017 179 B4 zu entnehmen.

Zwar gibt es heute schon akkumulatorbetriebene EMS-Reizerzeugungseinheiten, welche die über die EMS-Elektroden auf den Körper abzugebenden EMS-Reize formen und somit direkt am EMS-Kleidungsstück und damit am Körper "autark" getragen werden können, siehe WO 2011/089 263 A2 und WO 2014/000 736 A2 . In der Regel reicht die Akkuleistung jedoch nicht, um über einen im Trainingsbetrieb akzeptablen Zeitraum diese EMS-Reize zu erzeugen, welche aus Stromimpulsen und/oder Wechselstrom bestehen. Zu der Erzeugung dieser EMS-Reize weist die EMS-Reizerzeugungseinrichtung in der Regel einen elektrischen Impulsgenerator auf, sowie eine elektronische Steuerung, welche ein Anregungsschema vorgibt, dem folgend aus einem von einer Stromquelle bezogenen Strom eine zeitlich und auf die EMS-Elektroden des EMS-Trainingsgerätes verteilte Mehrzahl von EMS-Reizen geformt wird, die aus den Stromimpulsen und/oder Wechselstrom mit per Steuerung vorgegebenen Werten wie Amplitude und Frequenz bestehen, und mit denen die EMS-Elektroden beaufschlagt werden, um durch den Körper Strom mit vorgegebener Amplitude und Frequenzmuster durch zu leiten. Dabei sind die EMS-Elektroden an dem EMS-Kleidungsstück üblicherweise in Paaren gruppiert, so dass zwei zu einem solchen Paar gruppierte EMS-Elektroden jeweils über einen Leitungsast an der EMS-Reizerzeugungseinheit angeschlossen sind, die während des EMS-Trainings durch den Körper, an dem sie aufliegen, zu einem geschlossenen Stromkreis vervollständigt werden, der durch den Körper und somit durch die Muskeln hindurch führt.

Die die EMS-Elektroden mit der EMS-Reizerzeugungseinheit verbindenden Leitungsäste sind dabei zumindest zwischen einer Anschlussstelle am EMS-Kleidungsstück und der EMS-Reizerzeugungseinheit als EMS-Signalkabel ausgebildet, die von eine üblicherweise entfernt von dem Trainierenden und somit von den getragenen EMS-Kleidungsstücken angeordneten EMS-Reizerzeugungseinheit direkt zu den EMS-Elektroden oder zu entsprechenden Anschlüssen an den bzw. dem EMS-Kleidungsstück führen. Die EMS-Reizerzeugungseinheit ist dabei üblicherweise in einer eine Bedienoberfläche aufweisenden Steuereinheit, häufig in Form eines Steuerpults, untergebracht, und an das reguläre Stromnetz angeschlossen. Solche EMS-Trainingsgeräte werden von verschiedenen Herstellern produziert und professionell in Fitnessstudios von Personal-Trainern etc. betrieben, aber auch von Privatpersonen direkt. Die EMS-Elektroden sind mit der EMS-Reizerzeugungseinheit, also einem Steuergerät verschaltet, das beispielsweise in einem Frequenzbereich von 2 bis 150 Hz mit einer Impulsbreite von 50 bis 400 Mikrosekunden und einer Impulspause von 0 bis 10 Sekunden arbeitet. Der max. Scheitelwert der elektrischen Ausgangsspannung liegt beispielsweise bei 70 bis 160 Volt bei einer Stromstärke von ca. 10 bis 20 Milliampere.

Während die Erfolge der EMS-Methode beim Muskelaufbau unbestritten sind und EMS-Trainingsgeräte beim Publikum aufgrund der immer mehr verbesserten äußeren Erscheinung auch auf immer größeres Interesse stoßen, treten aufgrund des vermehrten Einsatzes auch systemische Probleme deutlicher zu Tage. So kommt es im Gebrauch zu unerklärlichen und zeitlich zufällig auftretenden Ausfällen von EMS-Elektroden, was bei den heutigen EMS-Kleidungsstücken, in die die Elektroden bereits integriert sind, zum Austausch des gesamten EMS-Kleidungsstücks, also beispielsweise einer Trainingsweste führt. Elektrodenausfall bedeutet in diesem Zusammenhang, dass die betroffene EMS-Elektrode keinen oder keinen ausreichenden Impulsstrom mehr an den Körper abgibt, obwohl keine Stromleitung oder Kontaktstelle zwischen der EMS-Elektrode und der die EMS-Elektrode mit Strom beaufschlagenden EMS-Reizerzeugungseinheit gebrochen und auch an der Elektrode äußerlich kein Defekt zu sehen sein muss.

Hiervon ausgehend ist es Aufgabe der vorliegenden Erfindung, eine EMS-Trainingsvorrichtung zu schaffen, mit denen die Funktionsfähigkeit der in der EMS-Trainingsvorrichtung eingesetzten EMS-Elektroden bzw. EMS-Kleidungsstücke über einen langen Zeitraum sichergestellt werden kann, sowie ein Verfahren zum Schutz einer EMS-Trainingsvorrichtung vor Verlust der Funktionsfähigkeit der in der EMS-Trainingsvorrichtung eingesetzten EMS-Elektroden bzw. EMS-Kleidungsstücke in dem Zeitraum.

Diese Aufgabe wird hinsichtlich der EMS-Trainingsvorrichtung mit den Merkmalen des Anspruchs 1 gelöst und hinsichtlich des Verfahrens mit den Merkmalen des Anspruchs 15.

Elektrostimulation ist ein komplexes elektrisches System. Getaktete Impulsströme werden von der von der EMS-Reizerzeugungseinheit erzeugt und über Stromleitungen zu den EMS-Elektroden und über die EMS-Elektroden in den Körper eingeleitet. Dabei werden im Regelfall die die EMS-Elektroden paarweise so geschaltet, dass bei einem Zeitpunkt die erste EMS-Elektrode mit dem Strom impulsartig beaufschlagt wird und die zweite EMS-Elektrode zu diesem Zeitpunkt nicht. Beim Impulswechsel wird die zweite EMS-Elektrode mit Strom impulsartig beaufschlagt und die erste EMS-Elektrode nicht. Da beim Training auf der Hautoberfläche Schweiß entsteht ist in dem Stromkreislauf EMS-Reizerzeugungseinheit (Steuergerät /Impulsstromversorgung), Stromleitung zur ersten EMS-Elektrode, erste EMS-Elektrode, Körper, zweite EMS-Elektrode, Stromleitung zurück zur EMS-Reizerzeugungseinheit zusätzlich der Elektrolyt Schweiß vorhanden. Der Stromlauf ist bei einer Bekleidung mit mehreren EMS-Elektrodenpaaren z.B. Brust, Bauch, Rücken Oberarme, Oberschenkel etc. noch komplexer.

Der Erfindung liegt die Erkenntnis zugrunde, dass in dem bzw. den Stromkreisen von der EMS-Reizerzeugungseinheit zu einer ersten EMS-Elektrode durch den Körper des Trainierenden hindurch über eine zweite EMS-Elektrode zurück zur EMS-Reizerzeugungseinheit unterschiedliche, auch temporär unterschiedliche Widerstände in dem von der EMS-Reizerzeugungseinheit über die erste EMS-Elektrode zum Körper führenden Leitungsast im Vergleich zu dem über die zweite EMS-Elektrode zurück zur EMS-Reizerzeugungseinheit führenden Leitungsast auftreten können.

Diese Widerstände werden durch sich ändernde zeitliche Gegebenheiten im Stromkreis, wie z.B. der unterschiedlich guten Anlage der jeweiligen EMS-Elektrode am Körper, lokal unterschiedlich starker Schweißabsonderung an den Hautoberflächen, unterschiedlich starker Nässung der beiden EMS-Elektroden etc. hervorgerufen, aber auch durch systemische Fehler, beispielsweise an den Verbindungsstellen der EMS-Elektroden mit EMS-Signalverbindungskabeln (Stromleitungen), die häufig als Druckknöpfe oder Crimp-Stecker ausgebildet sind.

Durch diese Widerstandsunterschiede kommt es zu unterschiedlichen Strömen im Strombeaufschlagungsleitungsast gegenüber dem Stromableitungsleitungsast, was zu einer Elektronenabgabe beispielsweise des Elektrodenmaterials, des Elektroden-/Stromleitungskontaktierungsmaterials oder in der Stromleitung und damit letztlich zu Oxidation führt.

Diese an unterschiedlichen Stellen im Stromkreis auftretende Oxidation und damit Korrosion, bei der an den Korrosionsstellen unter Elektronenabgabe das dort vorhandene metallische Material oxidiert und damit korrodiert, wirkt nun selbst so, als ob an den korrodierenden Stellen in elektrischer Hinsicht ein Zwischenwiderstand eingebaut würde. Mit der Zeit kann die Oxidation und damit Widerstandserhöhung soweit gehen, dass die im betroffenen Leitungsast angeordnete EMS-Elektrode keinen für die gewünschte Muskelkontraktion nötigen EMS-Reiz mehr übertragen kann. Durch den Schweiß bzw. die elektrolytische Wirkung des Schweißes wird dieser Vorgang verstärkt und beschleunigt.

Erfindungsgemäß wird nun vorgeschlagen, einen Unterschied des Widerstands in Zu- und Ableitung zu kompensieren, also auszugleichen, so dass es erst gar nicht zu einem weiteren, durch einsetzende Oxidation hervorgerufenen Widerstandsunterschied kommt und/oder dass ein durch einen trotzdem auftretenden Oxidations-Zwischenwiderstand hervorgerufener Widerstandsunterschied gleich wieder kompensiert wird. Dabei kann die Kompensation sowohl bei einem durch sich ändernde zeitliche Gegebenheiten im Stromkreis hervorgerufenen Widerstandsunterschied greifen, als auch bei einem wie vorstehend beschrieben durch einen systemischen Fehler hervorgerufenen Widerstandsunterschied.

Die erfindungsgemäße EMS-Trainingsvorrichtung weist also eine Kompensationseinrichtung auf, um in zumindest einem Leitungsast eine Abweichung des Widerstands von einem gewünschten Wert bzw. Sollwert zu kompensieren. Gemäß des erfindungsgemäßen Verfahrens zum Betrieb einer EMS-Trainingsvorrichtung wird bei einer Abweichung des Widerstands in zumindest einem Leitungsast von einem gewünschten Wert diese Abweichung kompensiert.

Erfindungsgemäß weist die EMS-Trainingsvorrichtung zum Schutz der EMS-Elektrode und/oder anderer Elemente zumindest eines der Leitungsäste vor Korrosion ferner zumindest eine Messeinrichtung auf, um in dem Leitungsast einen Widerstand oder eine mit dem Widerstand korrespondierende Größe als Istwert zu messen, welcher Istwert als Ausgangspunkt für einen Sollwert-Istwert-Vergleich dient, der wiederum Grundlage der vorstehend erläuterten Kompensation im Falle einer Abweichung des Istwerts vom Sollwert ist.

Erfindungsgemäß wird also ein Widerstand oder eine mit dem Widerstand korrespondierende Größe als Istwert gemessen. Diese gemessene Größe kann beispielsweise die aktuelle Impulsstromstärke bzw. der aktuelle elektrische Widerstand im jeweiligen Leitungsast sein. Es könnte aber auch eine Potentialmessung in den Impulspausen des EMS-Stromes erfolgen.

Zur Durchführung des Sollwert-Istwert-Vergleich weist die erfindungsgemäße EMS-Trainingsvorrichtung ferner eine Vergleichseinrichtung auf, um in dem jeweiligen Leitungsast den Istwert mit einem hinterlegten oder ermittelten Sollwert zu vergleichen. Entsprechend wird gemäß des erfindungsgemäßen Verfahrens der Istwert in dem jeweiligen Leitungsast mit einem hinterlegten oder ermittelten Sollwert verglichen.

Als Sollwerte können dabei in einem Speicher hinterlegte Sollwerte dienen, welche mit einer im zu kompensierenden Leitungsast gemessenen Stromstärke und/oder Spannung verglichen werden. Bevorzugt wird aber die während der jeweiligen Impulsstromgabe im jeweiligen Leitungsast erfasste Impulsstromstärke mit der im gepaarten Leitungsast erfassten Impulsstromstärke verglichen. Die Differenz der beiden erfassten Impulsstromstärken bildet dann den Istwert, wobei der Sollwert wiederum vorgegeben bzw. hinterlegt ist, bei identischen Leitungsästen also auf Null gesetzt ist. Falls nötig kann zusätzlich eine Spannungsmessung in den beiden Leitungsästen durchgeführt werden.

Als Sollwert dient dann die Impulsstromstärke des gepaarten Leitungsastes als mit dem dortigen Widerstand korrespondierende Größe. Die Vergleichseinrichtung kann den Sollwert für den zumindest einen Leitungsast also auf Null festlegen und die Abweichung des Istwerts vom Sollwert durch eine Subtraktion des Istwerts im jeweiligen Leitungsast vom Istwert in einem anderen Leitungsast, vorzugsweise dem gepaarten Leitungsast ermitteln. D.h., dass die Vergleichseinrichtung für den zumindest einen zu kompensierenden Leitungsast keinen festen Sollwert heranzieht, sondern die Abweichung des Istwerts vom Sollwert durch eine Subtraktion des Istwerts im jeweiligen Leitungsast vom Istwert in einem anderen Leitungsast, vorzugsweise dem gepaarten Leitungsast erfasst. Als Sollwert für einen der Leitungsäste kann auch ein Mittelwert der Istwerte in mehreren der Leitungsäste ermittelt werden bzw. für die Abweichung des Istwerts vom Sollwert eine Differenz des Istwerts im jeweiligen Leitungsast von dem Mittelwert der Istwerte in den anderen Leitungsästen.

Gemäß einer weiteren bevorzugten Ausführungsform wird dagegen als Istwert in den Zeitsegmenten der Impulspausen zwischen den EMS-Reizimpulsen, in denen kein Strom fließt, eine Potentialdifferenz zwischen dem jeweiligen Leitungsast und dem gepaarten Leitungsast erfasst, beispielsweise über automatische Potentialmessung. Das Potential bzw. die Potentialdifferenz zwischen den beiden Leitungsästen im Stromkreis entspricht einer elektrischen Ladung, die im Idealfall einen vorgegebenen bzw. vorgebbaren Sollwert annimmt, bei identischen Leitungsästen also Null sein soll (Sollwert). Der Istwert entspricht dann der erfassten bzw. gemessenen Potentialdifferenz, welche im Idealfall keine Abweichung vom vorgegebenen Sollwert hat, bei identischen Leitungsästen also Null ist.

Die Kompensation kann nun beispielsweise dadurch erfolgen, dass der Leitungsast mit dem höheren Widerstand mit einer entsprechend höheren Impulsspannung bzw. Impulsstrom beaufschlagt wird als der andere Leitungsast im Stromkreis. D.h., die EMS-Reize haben dann eine um die Kompensationsstromstärke -bzw. Spannung erhöhte Amplitude. Damit wird verhindert, dass eine EMS-Elektrode bzw. Kontaktierungsstelle zu viel Elektronen abgibt und nicht wieder auffüllen kann, also letztlich oxidiert/korrodiert. Die Kompensationseinrichtung für den zumindest einen Leitungsast kann dann also eine Spannungs- und/oder Stromstärkenerhöhungseinrichtung umfassen, über welche die Spannung- und/oder Stromstärke in dem Leitungsast bei einer Abweichung des Widerstands von einem gewünschten Widerstand also einer Abweichung eines Istwerts von einem Sollwert erhöht wird.

Die Kompensation kann auch durch einen in Zeitsegmenten der Impulspausen zwischen den EMS-Reizen im Anregungsschema dem zu kompensierenden Leitungsast zugeführten, gegenüber den Impulsen der EMS-Reizen vergleichsweise niedrigen, getakteten Strom erfolgen, der beispielsweise über die obenstehend erläuterte Potentialmessung in anderen Zeitsegmenten der Impulspausen geregelt wird.

Beispielsweise kann der Kompensationsstrom mit einer Stromstärke kleiner 2 mA und einer Spannung kleiner 5 V über 0,1 - 0,5 Sekunden getaktet jeweils in den nicht für die Messung genutzten Zeitsegmenten der Reizstrompausen zugeführt werden, wobei die Impulsdauer bei gängigen EMS-Trainingsgeräten ca. 0,2 bis 100 ms und die Dauer der Impulspause 0,1 bis 50 ms beträgt.

Die Kompensationseinrichtung kann für den zumindest einen der beiden Leitungsäste eine für die gewählte Kompensationsart geeignete Spannungs- und/oder Stromstärkenerhöhungseinrichtung aufweisen, so dass entweder die EMS-Reizimpulse erhöht werden oder die Impulspausen dazwischen bzw. diejenigen Zeitsegemente dieser Impulspausen, die auch nicht für eine Messung benötigt werden, für eine Erhöhung der Spannung- und/oder Stromstärke im jeweiligen Leitungsast genutzt werden. Es wäre aber auch denkbar, den Kompnesationsstrom bzw. -spannung sowohl den EMS-Reizen zu überlagern als auch in den nicht für Messzwecke genutzten Zeitsegmenten der Impulspausen zuzuführen.

Bevorzugt weist die Kompensationseinrichtung für den zu kompensierenden, einen der beiden Leitungsäste eine Spannungs- und/oder Stromstärkenerhöhungseinrichtung auf, über welche die Spannung- und/oder Stromstärke in dem Leitungsast bei einer Abweichung der gemessenen Potentialdifferenz (Istwert) von der gewünschten Potentialdifferenz (Sollwert) zwischen den beiden Leitungsästen der gepaarten EMS- Elektroden, also bei einer Abweichung des Istwerts vom Sollwert erhöht wird. Der zwischen den EMS-Reizen zugeführte, gegenüber den Impulsen der EMS-Reizen vergleichsweise niedrige, getaktete Strom sollte so niedrig sein, dass er vom EMS-Trainierenden möglichst nicht spürbar ist und auch nicht die Muskelstimulation stört.

Alternativ oder ergänzend dazu kann die Kompensationseinrichtung für den zumindest einen Leitungsast auch eine an oder in der Nähe der EMS-Elektrode dieses Leitungsasts angebrachte und über eine separate Leitung mit einer Stromquelle - im Regelfall also mit der als Stromquelle fungierenden EMS-Reizerzeugungseinheit - verbundene Schutzelektrode aufweisen, welche bei einer Abweichung des Istwerts vom Sollwert mit Strom beaufschlagt wird. Die Schutzelektrode wird durch die Strombeaufschlagung zur Elektronenabgabe gezwungen. Diese Elektronen ersetzten dann diejenigen Elektronen, die die oxidationsgefährdete EMS-Elektrode sonst abgeben würde. Somit wird also der Oxidationsprozess der EMS-Elektrode aufgehalten oder zumindest verlangsamt.

Die zu der bzw. den Schutzelektroden gesandten Kompensationsströme und die dazugehörigen Spannungen sollten dabei allerdings ebenfalls so niedrig sein, dass sie vom EMS-Trainierenden nicht oder nicht störend wahrgenommen werden und die Muskelstimulation nicht behindern. Zur Kompensation des erhöhten Widerstands im jeweiligen Leitungsast kann die Schutzelektrode vorteilhaft in Zeitsegmenten der Impulspausen zwischen den EMS-Reizen mit einem gegenüber den Impulsen der EMS-Reizen vergleichsweise niedrigen, getakteten Strom beaufschlagt werden. Dieser getaktete Kompensationsstrom kann dabei ebenfalls über eine Erfassung der Potentialdifferenz zwischen dem zu kompensierenden Leitungsast und dem gepaarten Leitungsast erfolgen. Alternativ kann die Messung über eine Erfassung der Potentialdifferenz zwischen dem zu kompensierenden Leitungsast und dem Leitungsast mit der zugeordneten Schutzelektrode geregelt werden. In diesem Fall fungiert die Schutzelektrode bzw. der Leitungsast mit der Schutzelektrode auch als Teil der Messvorrichtung. In beiden Fällen wird die Messung vorteilhaft in den nicht für die Kompensationsstrombeschickung verwendeten Zeitsegmenten der Impulspausen automatisch durchgeführt.

Beispielsweise kann der Kompensationsstrom mit einer Stromstärke kleiner 2 mA und einer Spannung kleiner 5 V über 0,1 - 0,5 Sekunden getaktet jeweils in den nicht für die Messung genutzten Zeitsegmenten der Reizstrompausen zugeführt werden, wobei die Impulsdauer bei gängigen EMS-Trainingsgeräten ca. 0,2 bis 100 ms und die Dauer der Impulspause 0,1 bis 50 ms beträgt.

Bei der Anordnung der Schutzelektrode direkt auf der EMS-Elektrode ist eine Isolationsschicht zwischen Schutzelektrode und EMS-Elektrode erforderlich. Damit wird eine für die Regelung geeignete Potentialmessung ermöglicht. Der Elektronenersatz erfolgt bei der Beaufschlagung eines Kompensationstromes an der Schutzelektrode über die feuchte bzw. Schweißumgebung an den beiden Elektroden.

Die Schutzelektrode(n) kann bzw. können aus beschichtetem Titan oder einer beschichteten Titanlegierung oder aus Edelmetall bestehen. Die Beschichtung kann aus Mischoxiden oder aus Edelmetall bestehen oder Mischoxide oder Edelmetall enthalten. Dadurch gelingt es, dass die Schutzelektroden selbst nicht oder nur schwach oxidieren.

Das erfindungsgemäße Elektrodenschutzverfahren kann dabei von einem Techniker von Fall zu Fall durchgeführt werden, etwa im Rahmen einer wöchentlichen oder monatlichen Wartung der EMS-Trainingsvorrichtung. Er kann dann mit der Messeinrichtung die Messung der Widerstands-Istwerte vornehmen, entweder über eine vorhandene Vergleichseinrichtung oder im Kopf den Vergleich mit den Sollwerten vornehmen und dann mittels der Kompensationseinrichtung die Kompensation, also beispielsweise eine Stromstärkenerhöhung per Drehregler für einen betroffenen Leitungsast vornehmen. Bevorzugt weist die EMS-Trainingsvorrichtung jedoch eine übergeordnete Steuerung auf, welche im Ansprechen auf eine erfasste Abweichung des Istwerts vom Sollwert in zumindest einem der Leitungsäste den dortigen Istwert auf den dortigen Sollwert steuert oder bevorzugt regelt, so dass auf eine Abweichung automatisch reagiert und damit die Lebensdauer der EMS-Elektroden und der damit versehenen EMS-Kleidungsstücke maximal erhöht wird. Es wäre im Rahmen der Erfindung auch denkbar, dass die übergeordnete Steuerung, die Messeinrichtung, die Vergleichseinrichtung und die Kompensationseinrichtung in einem separaten, an die EMS-Trainingsvorrichtung anschließbaren Gerät angeordnet sind.

Es wäre ferner denkbar, anstatt oder ergänzend zu den Schutzelektroden jeder EMS-Elektrode bzw. jedem von der EMS-Reizerzeugungseinheit zur jeweiligen EMS-Elektrode führenden Leitungsast eine sich selbst verbrauchende Opferanode zuzuordnen. D.h. anstatt einer nicht korrodierenden Schutzelektrode eine Opferanode mit dem jeweiligen Leitungsast elektrisch leitend zu verbinden. Es wäre auch denkbar, eine gemeinsame Opferanode für mehrere Leitungsäste vorzusehen und mit mehreren Leitungsästen leitend zu verbinden, beispielsweise mit solchen, die gleichgetaktet mit EMS-Reizen, also beispielsweise Stromimpulsen beschickt werden, oder sogar - mit entsprechender Verkabelung bzw. entsprechender Verschaltung - sternförmig mit allen Leitungsästen. Möglicherweise wäre es aufgrund der komplexen Stromflüsse bei einer heutigen EMS-Trainingsvorrichtung mit einer Vielzahl von EMS-Elektroden sogar möglich, den vorstehend beschriebenen Korrosionsschutzeffekt zu erzielen, ohne dass alle Leitungsäste selbst direkt mit einer Opferanode in Verbindung stehen.

Während man bei der Wahl des Materials für die Opferanode auf ein Material mit einem negativeren Standardelektrodenpotential als das für die elektrisch leitenden Teile der EMS-Elektrode und die elektrisch leitende Verbindung zwischen der EMS-Reizerzeugungseinheit und der EMS-Elektrode verwendete Material festgelegt ist, also in der Regel auf ein Material und insbesondere ein Metall mit einem negativen Standardelektrodenpotential in der elektrochemischen Spannungsreihe (aus Kosten- und Verarbeitungsgründen bevorzugt Magnesium, aus Hautverträglichkeitsgründen möglichst kein Buntmetall) ist man in der Wahl des Anbringungsorts der Opferanode weitgehend frei, solange diese mit dem zu schützenden EMS-Elektroden bzw. Leitungsästen in Verbindung steht bzw. stehen.

Vorteilhaft sind die EMS-Elektroden an einem oder mehreren EMS-Kleidungsstücken, wie z.B. Jacke, Gürtel, Hose, Arm- und/oder Beinband angeordnet insbesondere aufgenäht oder im Flachstrickverfahren eingestrickt. Von den EMS-Kleidungsstücken ist vorteilhaft zumindest ein Teil als Textilie ausgebildet, wobei das jeweilige EMS-Kleidungsstück für die EMS-Elektrode vorgesehene Schutzelektroden tragen kann.

Vorteilhaft sind die EMS-Reizerzeugungseinheit, die übergeordnete Steuerung , die Vergleichseinrichtung, vorzugsweise die Messeinrichtung und vorzugsweise die Kompensationseinrichtung in einem gemeinsamen, vorzugsweise als Steuerpult ausgebildeten Gehäuse angeordnet, welches einen Anschluss an das Stromnetz aufweist.

In den beiliegenden Figuren werden verschiedene Ausführungsformen in der Erfindung näher erläutert. Es zeigen:
- Figur 1: eine schematische Ansicht einer EMS-Trainingsvorrichtung gemäß einer ersten Ausführungsform der Erfindung;
- Figur 2: eine schematische Ansicht einer EMS-Trainingsvorrichtung gemäß einer weiteren Ausführungsform der Erfindung.

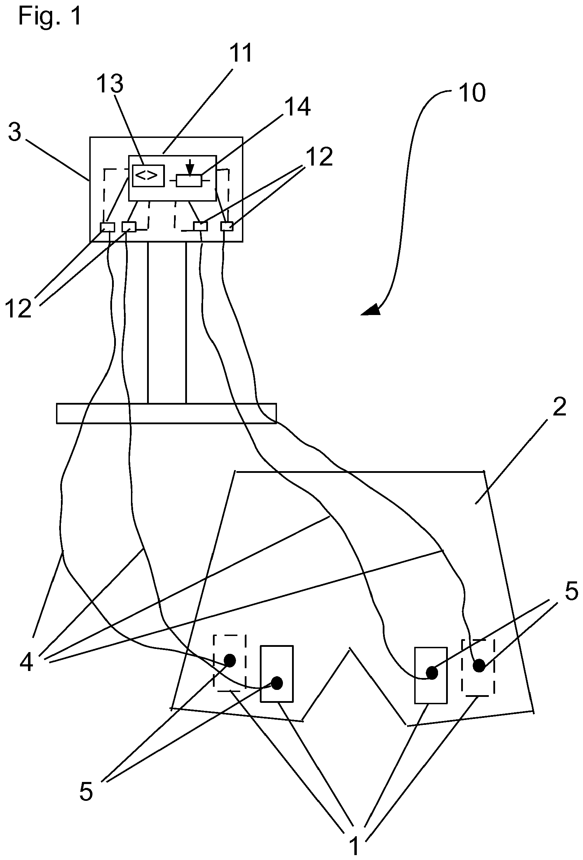

In der Figur 1 ist ein als kurze Hose gestaltetes EMS-Kleidungsstück 2 gezeigt, welches mit vier jeweils zu zwei Paaren gruppierten EMS-Elektroden 1 bestückt ist, wobei jedes Elektrodenpaar einem Oberschenkel zugeordnet ist, und wobei jede EMS-Elektrode 1 über ein Signalkabel bzw. einen Leitungsast 4 mit einer in ein pultförmiges Steuergerät eingebauten EMS-Reizerzeugungseinheit 3 verbunden ist. Das EMS-Kleidungsstück könnte natürlich auch eine andere Gestalt haben, etwa eine Weste oder ein Ganzkörperanzug sein. Es könnte auch ein Satz von mehreren EMS-Kleidungsstücken, z.B. Hose, Weste, Armbänder, Unterschenkelbänder vorgesehen sein. Die EMS-Elektroden 1 können dabei Druckknöpfe 5 aufweisen, an denen die die zu den EMS-Elektroden 1 führenden Leitungsäste bildenden Signalkabel 4 mit entsprechenden Steckern aufgesteckt sind. Andere Verbindungsarten wären denkbar.

In dem Steuerpult mit der EMS-Reizerzeugungseinheit 3, welche aus einem Netzstrom EMS-Reize gemäß eines gewünschten Anregungsschema formt, sind eine Messeinrichtung 12 bildende Messsensoren integriert, wobei jedem Leitungsast 4 einer der Messsensoren zugeordnet ist, welcher eine Amplitude einer Impulsstromstärke in dem jeweiligen Leitungsast 4 erfasst. In dem Steuerpult ist ferner eine übergeordnete Steuerung 11 integriert, welche für in jeden der Leitungsäste 4 im Ansprechen auf eine erfasste Abweichung des Istwerts der Impulsstromstärke von einem Sollwert der dortigen Impulsstromstärke den dortigen Istwert auf den dortigen Sollwert automatisch regelt.

Dazu weist die Steuerung 11 eine Vergleichseinrichtung 13 auf, etwa in Form einer fachnotorisch bekannten Vergleicherschaltung, über welche der Sollwert-Istwert-Vergleich durchgeführt wird, sowie eine Kompensationseinrichtung 14, um für jeden Leitungsast 4 eine Abweichung des Istwerts vom Sollwert zu kompensieren, etwa die Amplitude der Spannungs- oder Stromimpulse in dem betroffenen Leitungsast zu erhöhen. Die Kompensationseinrichtung 14 kann für jeden Leitungsast 4 separat durch ein Regelglied in einem geschlossenen Regelkreis ausgebildet sein. Die Vergleichseinrichtung 13 und die Kompensationseinrichtung 14 können in einer gemeinsamen Schaltung zusammengefasst sein. Die Kompensationseinrichtung könnte auch in Form von Drehreglern zur händischen Nachstellung vorgesehen sein, wobei dann eine Anzeige oder Ausgabe für eine erfasste Abweichung vorhanden sein müsste.

Als Sollwert für den Sollwert-Istwert-Vergleich für jeden Leitungsast 4 wird dabei die im gepaarten Leitungsast 4 während der dortigen Impulsstromgabe gemessene Impulsstromstärke verwendet, also in demjenigen Leitungsast, der mit dem zu überprüfenden Leitungsast 4 einen geschlosssenen Stromkreis von der EMS-Reizerzeugungseinheit 3 zu einer ersten EMS-Elektrode 1, durch den Oberschenkel hindurch zu einer zweiten EMS-Elektrode 1 und zurück zur EMS-Reizerzeugungseinheit 3 bildet. Ergibt sich eine Differenz, bzw. wird ein Differenzschwellwert überschritten, wird nachgeregelt. Alternativ zur Messung und Regelung der Impulsstromstärke könnte in den Zeitsegmenten der Impulspausen zwischen den EMS-Reizimpulsen ein sehr niedrig getakteter Strom zugeführt werden, der über automatische Potentialmessung in anderen Zeitsegmenten der Impulspausen geregelt wird.

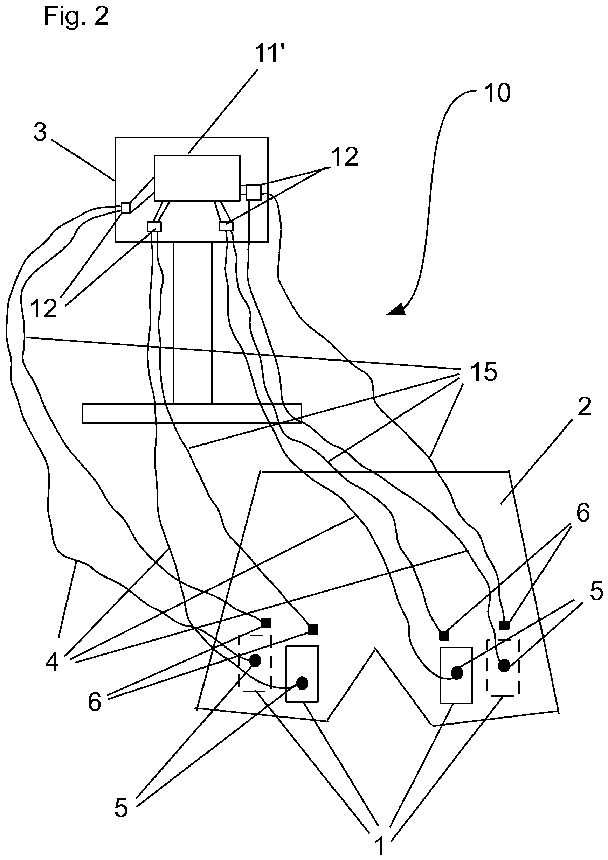

Eine weitere Ausführungsform der Erfindung ist in Fig. 2 gezeigt. Als Kompensationseinrichtung für die Kompensation einer Abweichung eines widerstandskorrespondieren Istwerts vom Sollwert in den einzelnen Leitungsästen ist dabei in jedem zu einer der EMS-Elektroden 1 führenden Leitungsast 4 ein weiterer Ausgang an dem Steuerpult mit der EMS-Reizerzeugungseinheit 3 und der Steuerung 11' zugeordnet, welcher Ausgang mit einer Leitung zu einer Schutzelektrode 6 in der Nähe der jeweiligen EMS-Elektrode 1 verbunden ist. Diese Schutzelektrode 6 kann aus beschichtetem Titan oder einer beschichteten Titanlegierung oder aus Edelmetall bestehen, wobei die Beschichtung aus Mischoxiden oder Edelmetall sein kann. Von der Steuerung 11' wird die jeweilige Schutzelektrode 6 in Zeitsegmenten der Impulspausen zwischen den EMS-Reizimpulsen mit einem getakteten Strom beaufschlagt, der über automatische Potentialmessung in anderen Zeitsegmenten der Impulspausen geregelt wird.

Diese kleinen Elektroden bzw. Schutzelektroden 6 sollen dabei ohne selbst zu oxidieren Elektronen angeben (durch die Strombeaufschlagung) und damit den Oxidationsprozess der oxidationsgefährdeten EMS-Elektroden 4 aufhalten oder soweit verlangsamen, dass das EMS-Kleidungsstück 2 seine wirtschaftliche Lebensdauer erreicht. Die Ströme und die dazugehörige Spannung müssen so niedrig sein, dass sie vom EMS-Trainierenden nicht wahrgenommen werden und die Muskelstimulation nicht behindern.

Abwandlungen und Modifikationen der gezeigten Ausführungsformen sind möglich, ohne den Rahmen der Erfindung zu verlassen.

## Patentansprüche

1. EMS-Trainingsvorrichtung (10), mit
einer EMS-Reizerzeugungseinheit (3), welche aus einem von einer Stromquelle bezogenen Strom eine einem vorgegebenen Anregungsschema folgende, zeitlich und elektrodenmäßig verteilte Mehrzahl von EMS-Reizen formt, die aus Stromimpulsen und/oder Wechselstrom mit vorgegebenen Werten wie Amplitude und Frequenz bestehen,
insbesondere paarweise einander zugeordnet an einem lebenden Körper anbringbaren EMS-Elektroden (1) zur Beaufschlagung des Körpers mit EMS-Reizen,
Leitungsästen (4), wobei jeder Leitungsast (4) eine der EMS-Elektroden (1) elektrisch leitend mit der EMS-Reizerzeugungseinheit (3) verbindet, um diese EMS-Elektrode (1) mit den ihr zugeordneten EMS-Reizen zu beaufschlagen, so dass durch den Körper Strom mit vorgegebener Amplitude und Frequenzmuster durchgeleitet wird, insbesondere in einer Anzahl von jeweils zwei der Leitungsäste (4) umfassenden Stromkreisen, die jeweils zwei paarweise einander zugeordnete EMS-Elektroden (1) aufweisen,
**dadurch gekennzeichnet, dass**
die EMS-Trainingsvorrichtung (10) zum Korrosionsschutz der EMS-Elektrode (1) und/oder anderer dafür anfälliger Elemente (5) zumindest eines der Leitungsäste (4), zumindest eine Messeinrichtung (12) aufweist, um in dem Leitungsast (4) einen Widerstand oder eine mit dem Widerstand korrespondierende Größe als Istwert zu erfassen, zumindest eine Vergleichseinrichtung (13), um den Istwert in dem Leitungsast (4) mit einem hinterlegten oder ermittelten Sollwert zu vergleichen, und zumindest eine Kompensationseinrichtung (14; 6, 15), um in dem Leitungsast (4) eine Abweichung des Istwerts vom Sollwert zu kompensieren.

2. EMS-Trainingsvorrichtung (10) nach Anspruch 1, **dadurch gekennzeichnet, dass** die EMS-Trainingsvorrichtung (10) eine übergeordnete Steuerung (11; 11') aufweist, welche im Ansprechen auf ein Feststellen einer Abweichung des Istwerts vom Sollwert in zumindest einem der Leitungsäste (4) den dortigen Istwert auf den dortigen Sollwert steuert oder regelt.

3. EMS-Trainingsvorrichtung (10) nach Anspruch 1 oder 2, **dadurch gekennzeichnet, dass** die EMS-Trainingsvorrichtung (10) einen Speicher umfasst, in dem der Sollwert für den zumindest einen Leitungsast (4) abgelegt ist.

4. EMS-Trainingsvorrichtung (10) nach Anspruch 1, 2 oder 3, **dadurch gekennzeichnet, dass** die Istwert-Erfassung durch eine Subtraktion des erfassten Istwerts im jeweiligen Leitungsast (4) vom erfassten Istwert in einem anderen Leitungsast (4) oder einem Mittelwert der erfassten Istwerte in mehreren der Leitungsäste (4) erfolgt, insbesondere durch eine Substraktion des Istwerts im jeweiligen Leitungsast (4) vom Istwert in demjenigen Leitungsast (4), der von der EMS-Reizerzeugungseinheit (3) zu derjenigen EMS-Elektrode (1) führt, die mit der in dem zu kompensierenden Leitungsast (4) angeordneten EMS-Elektrode (1) ein Paar in einem Stromkreis durch den lebenden Körper einander zugeordneter EMS-Elektroden (1) bildet, wobei die Vergleichseinrichtung (13) den Sollwert für den zumindest einen zu kompensierenden Leitungsast (4) bevorzugt auf Null festlegt.

5. EMS-Trainingsvorrichtung (10) nach einem der vorhergehenden Ansprüche, **dadurch gekennzeichnet, dass** die Istwert-Erfassung durch Messung einer Potentialdifferenz zwischen zwei Leitungsästen (4) mit gepaarten EMS-Elektroden in solchen Zeitsegmenten der Impulspausen zwischen den EMS-Reizen im Anregungsschema erfolgt, in denen in den beiden Leitungsästen (4) keine Kompensations-Stromzufuhr stattfindet.

6. EMS-Trainingsvorrichtung (10) nach einem der vorhergehenden Ansprüche, **dadurch gekennzeichnet, dass** die Kompensationseinrichtung (14) für den zumindest einen Leitungsast (4) eine Spannungs- und/oder Stromstärkenerhöhungseinrichtung (14) umfasst, über welche die Spannung- und/oder Stromstärke in dem Leitungsast (4) bei einer Abweichung des Istwerts vom Sollwert - im Ansprechen auf eine Benutzereingabe oder durch die Steuerung (11) - erhöht wird.

7. EMS-Trainingsvorrichtung (10) nach Anspruch 6, **dadurch gekennzeichnet, dass** die Kompensation durch eine Erhöhung der Amplituden der Stromstärke und/oder Spannung der EMS-Reize erfolgt.

8. EMS-Trainingsvorrichtung (10) nach Anspruch 6 oder 7, **dadurch gekennzeichnet, dass** die Kompensation in solchen Zeitsegmenten der Impulspausen zwischen den EMS-Reizen im Anregungsschema erfolgt, in denen in dem jeweiligen zu kompensierenden Leitungsast (4) keine Messung durchgeführt wird, wobei die Kompensation durch einen dem jeweiligen, zu kompensierenden Leitungsast (4) zugeführten, gegenüber den Amplituden der Stromstärke der EMS-Reize niedrigeren, getakteten Strom erfolgt.

9. EMS-Trainingsvorrichtung (10) nach einem der vorhergehenden Ansprüche, **dadurch gekennzeichnet, dass** die Kompensationseinrichtung (14) für den zumindest einen Leitungsast (4) eine an oder in der Nähe der EMS-Elektrode (1) dieses Leitungsasts (4) angebrachte und über eine separate Leitung (15) mit einer Stromquelle verbundene Schutzelektrode (6) aufweist, welche bei einer Abweichung des Istwerts vom Sollwert - im Ansprechen auf eine Benutzereingabe oder durch die Steuerung / Regelung (11') - mit Strom beaufschlagt wird.

10. EMS-Trainingsvorrichtung (10) nach Anspruch 9, **dadurch gekennzeichnet, dass** die Istwert-Erfassung durch Messung einer Potentialdifferenz zwischen dem jeweiligen, zu kompensierenden Leitungsast (4) und der seiner EMS-Elektrode (1) zugeordneten Schutzelektrode (6) in solchen Zeitsegmenten der Impulspausen zwischen EMS-Reizen im Anregungsschema erfolgt, in denen in dem jeweiligen Leitungsast (4) keine Kompensations-Stromzufuhr stattfindet.

11. EMS-Trainingsvorrichtung (10) nach Anspruch 9 oder 10, **dadurch gekennzeichnet, dass** die Kompensation durch eine Regelung der Spannung und/oder Stromstärke in dem jeweiligen Leitungsast (4) bei einer Abweichung der Potentialdifferenz von einer gewünschten Potentialdifferenz zwischen diesem Leitungsast (1) und der zugeordneten Schutzelektrode (6) erfolgt.

12. EMS-Trainingsvorrichtung (10) nach einem der Ansprüche 9 bis 11, **dadurch gekennzeichnet, dass** die Schutzelektrode (6) aus einem Edelmetall, einer Edelmetalllegierung, aus mit Edelmetall oder Mischoxiden beschichtetem Titan oder einer mit Edelmetall oder Mischoxiden beschichteten Titanlegierung besteht, wobei bei einer vorteilhaften Anordnung der Schutzelektrode (6) direkt auf der zugeordneten EMS-Elektrode (1) zwischen der Schutzelektrode (6) und der EMS-Elektrode (1) eine Isolationsschicht vorhanden ist.

13. EMS-Trainingsvorrichtung (10) nach einem der vorhergehenden Ansprüche, **dadurch gekennzeichnet, dass** in jedem der Leitungsäste (4) die Abweichung des Istwerts vom Sollwert auf Basis eines gemessenen oder ermittelten Widerstands bzw. einer gemessenen oder ermittelten, mit dem Widerstand korrespondierenden Größe im Ansprechen auf eine Benutzereingabe oder durch die Steuerung (11; 11') kompensiert wird, wobei vorteilhaft für jeden Leitungsast zumindest eine eigene Messeinrichtung (12), bevorzugt auch eine eigene Kompensationseinrichtung (14; 6, 15) und/oder eine Vergleichseinrichtung (13) vorgesehen ist.

14. EMS-Trainingsvorrichtung (10) nach einem der vorhergehenden Ansprüche, **dadurch gekennzeichnet, dass** die EMS-Elektroden (1) jeweils ein sich flexibel an den Körper anlegendes, flächiges Pad oder einen Textilstrukturabschnitt mit einer elektrisch leitenden Leitschicht aufweist, insbesondere mit einer metallische oder metallisierte Fäden enthaltenden Leitschicht, die EMS-Elektroden (1) jeweils eine Schutzelektrode (6) tragen, welche mit der EMS-Reizerzeugungseinheit (3) elektrisch leitend verbunden oder verbindbar ist.

15. Verfahren zum Schutz einer EMS-Trainingsvorrichtung (10) vor durch Betrieb der EMS-Trainingsvorrichtung (10), insbesondere einer EMS-Trainingsvorrichtung (10) nach einem der vorhergehenden Ansprüche hervorgerufener Korrosion einer EMS-Elektrode (1) und/oder anderer für Korrosion anfälliger Elemente (5), wie z.B. eines Druckknopfs, in zumindest einem Leitungsast (4), wobei im Betrieb der EMS-Trainingsvorrichtung (10) deren am lebenden Körper anbringbare EMS-Elektroden (1) jeweils elektrisch leitend an deren EMS-Reizerzeugungseinheit (3) angeschlossen sind, um Leitungsäste (4) zu bilden, die während des EMS-Trainings durch den Körper hindurch zu einem oder mehreren geschlossenen Stromkreisen vervollständigt werden, wobei insbesondere jeweils ein Paar der Leitungsäste (4) einander zugeordnet sind, um während des EMS-Trainings einen durch den Körper verlaufende, geschlossenen Stromkreis zu bilden,
**dadurch gekennzeichnet, dass** ein Widerstand oder eine mit dem Widerstand korrespondierende Größe als Istwert gemessen wird, der Istwert in dem Leitungsast (4) mit einem hinterlegten oder ermittelten Sollwert verglichen wird, und bei einer Abweichung des Istwerts vom Sollwert die Abweichung kompensiert wird, wobei insbesondere gemäß der Merkmale eines der Ansprüche 4, 5, 7, 8, 11, 12 vorgegangen wird.

## Claims

1. An EMS training device (10), comprising
an EMS stimulation generating unit (3) which forms from a current drawn from a current source a plurality of EMS stimuli which follows a predetermined stimulation diagram and is distributed in respect of time and electrodes, and which consist of current pulses and/or alternating-current with predetermined values such as amplitude and frequency,
EMS electrodes (1) attachable to a living body, in particular pairwise associated with each other for the purpose of applying the stimuli to the body,
line branches (4), wherein each line branch (4) connects one of the EMS electrodes (1) in an electrically conductive manner to the EMS stimulus generating unit (3) in order to apply said EMS electrode (1) with EMS stimuli associated with said electrode, so that current with a predetermined amplitude and frequency pattern is conducted through the body, in particular in a number of current circuits respectively comprising two of the line branches (4), which circuits respectively comprise two EMS electrodes (1) associated with each other in pairs,
**characterized in that**
the EMS training device (10) comprises, for corrosion protection of the EMS electrode (1) and/or other elements susceptible thereto of at least one of the line branches (4), at least one measuring device (12) in order to detect in the line branch (4) a resistance or a quantity corresponding to the resistance as an actual value, at least one comparison device (13) in order to compare the actual value in the line branch (4) with a stored or determined target value, and at least one compensation device (14; 6, 15) in order to compensate a deviation of the actual value from the target value in the line branch (4).

2. An EMS training device (10) according to claim 1, **characterized in that** the EMS training device (10) comprises a higher-level controller (11; 11'), which, in response to a determination of a deviation of the actual value from the target value in at least one of the line branches (4), controls or feedback controls the local actual value to the local target value.

3. An EMS training device (10) according to claim 1 or 2, **characterized in that** the EMS training device (10) comprises a memory in which the target value for the at least one line branch (4) is stored.

4. An EMS training device (10) according to claim 1, 2 or 3, **characterized in that** the actual value detection occurs by a subtraction of the detected actual value in the respective line branch (4) from the detected actual value in another line branch (4) or a mean value of the detected actual values in several of the line branches (4), especially by a subtraction of the actual value in the respective line branch (4) from the actual value in the line branch (4) which leads from the EMS stimulus generating unit (3) to those EMS electrode (1), which together with the EMS electrode (1) arranged in the line branch (4) to be compensated forms a pair in a current circuit through the living body of mutually associated EMS electrodes (1), wherein the comparison device (13) preferably fixes the target value for the at least one line branch (4) to be compensated to zero.

5. An EMS training device (10) according to one of the preceding claims, **characterized in that** the actual value detection occurs by measurement of a potential difference between two line branches (4) with paired EMS electrodes in such time segments of the interpulse periods between the EMS stimuli in the stimulation diagram, in which no compensation current feed occurs in the two line branches (4).

6. An EMS training device (10) according to one of the preceding claims, **characterized in that** the compensation device (14) for the at least one line branch (4) comprises a voltage and/or current intensity increasing device (14), via which, in response to a user input or by the controller (11), the voltage and/or current intensity in the line branch (4) is increased upon a deviation of the actual value from the target value.

7. An EMS training device (10) according to claim 6, **characterized in that** the compensation occurs by an increase in the amplitudes of the current intensity and/or the voltage of the EMS stimuli.

8. An EMS training device (10) according to claim 6 or 7, **characterized in that** the compensation occurs in such time segments of the interpulse periods between the EMS stimuli in the stimulation diagram in which no measurement is carried out in the respective line branch (4) to be compensated, wherein the compensation occurs by a clocked current which is fed to the respective line branch (4) to be compensated and which is lower in relation to the amplitudes of the current intensity of the EMS stimuli.

9. An EMS training device (10) according to one of the preceding claims, **characterized in that** the compensation device (14) for the at least one line branch (4) comprises a protective electrode (6) which is attached or close to the EMS electrode (1) of said line branch (4), is connected via a separate line (15) to a current source and, in response to a user input or by the controller/feedback controller (11'), is fed with current upon deviation of the actual value from the target value.

10. An EMS training device (10) according to claim 9, **characterized in that** the actual value detection occurs by measurement of a potential difference between the respective line branch (4) to be compensated and the protective electrode (6) associated with its EMS electrode (1) in such time segments of the interpulse periods between the EMS stimuli in the stimulation diagram, in which no compensation current feed occurs in the respective line branch (4).

11. An EMS training device (10) according to claim 9 or 10, **characterized in that** the compensation occurs by a feedback control of the voltage and/or current intensity in the respective line branch (4) in the event of a deviation of the potential difference from a desired potential difference between said line branch (4) and the associated protective electrode (6).

12. An EMS training device (10) according to one of the claims 9 to 11, **characterized in that** the protective electrode (6) consists of a noble metal, a noble metal alloy, titanium coated with a noble metal or mixed oxides, or a titanium alloy coated with noble metal or mixed oxides, wherein in an advantageous arrangement of the protective electrode (6) an insulation layer is directly present on the associated EMS electrode (1) between the protective electrode (6) and the EMS electrode (1).

13. An EMS training device (10) according to one of the preceding claims, **characterized in that** in each of the line branches (4), in response to a user input or an input by the controller (11; 11'), the deviation of the actual value from the target value is compensated on the basis of a measured or determined resistance or a measured or determined quantity corresponding to the resistance, wherein advantageously for each line branch at least one measuring device (12) is provided, preferably also a separate compensation device (14; 6, 15) and/or a comparison device (13).

14. An EMS training device (10) according to one of the preceding claims, **characterized in that** the EMS electrodes (1) each comprise a flat pad which rests flexibly on the body or a textile structure section with an electrically conductive conducting layer, especially with a conductive layer containing metallic or metallised threads, the EMS electrodes (1) each carry a protective electrode (6) which is or can be connected in an electrically conductive manner to the EMS stimulus generating unit (3).

15. A method for the protection of an EMS training device (10) from corrosion of an EMS electrode (1) and/or other elements (5) susceptible to corrosion such as a push button in at least one line branch (4), which corrosion is caused by the operation of the EMS training device (10), especially an EMS training device (10) according to one of the preceding claims, wherein in the operation of the EMS training device (10) its EMS electrodes (1) that can be attached to the living body are each electrically conductively connected to its EMS stimulus generating unit (3) in order to form line branches (4), which during the EMS training are completed through the body to form one or several closed current circuits, wherein especially one pair each of the line branches (4) are associated with each other in order to form a closed current circuit extending through the body during the EMS training, **characterized in that** a resistance or a quantity corresponding to the resistance is measured as an actual value, the actual value is compared in the line branch (4) with a stored or determined target value, and in the event of a deviation of the actual value from the target value the deviation is compensated, wherein it is proceeded especially according to the features of one of the claims 4, 5, 7, 8, 11, 12.

## Revendications

1. Dispositif d'entraînement ESM (10) comprenant
une unité de génération de stimuli ESM (3) qui forme, à partir d'un courant obtenu d'une source de courant, une pluralité de stimuli ESM qui suivent un schéma d'excitation prédéfini et sont répartis dans le temps et par électrode et qui sont constitués par des impulsions de courant et/ou du courant alternatif présentant des valeurs prédéfinies telles que l'amplitude et la fréquence,
des électrodes ESM (1) qui peuvent être montées sur un corps vivant, en étant associées en particulier par paires les unes aux autres, et qui sont destinées à soumettre le corps à des stimuli ESM,
des branches de ligne (4), chaque branche de ligne (4) reliant de manière électroconductrice l'une des électrodes ESM (1) à l'unité de génération de stimuli ESM (3), afin de soumettre cette électrode ESM (1) aux stimuli ESM qui lui sont associés, de sorte qu'un courant d'une amplitude et d'un motif de fréquence prédéfinis est amené à traverser le corps, en particulier dans un nombre de circuits qui comprennent respectivement deux des branches de ligne (4) et présentent chacun deux électrodes ESM (1) associées l'une à l'autre par paire,
**caractérisé par le fait que**
ledit dispositif d'entraînement ESM (10) comprend, pour protéger contre la corrosion l'électrode ESM (1) et/ou d'autres éléments (5) sensibles d'au moins l'une des branches de ligne (4), au moins un dispositif de mesure (12) afin de saisir dans la branche de ligne (4), en tant que valeur réelle, une résistance ou une grandeur correspondant à la résistance, au moins un dispositif de comparaison (13) afin de comparer la valeur réelle dans la branche de ligne (4) à une valeur de consigne mise en mémoire ou déterminée, et au moins un dispositif de compensation (14 ; 6, 15) afin de compenser dans la branche de ligne (4) un écart entre la valeur réelle et la valeur de consigne.

2. Dispositif d'entraînement ESM (10) selon la revendication 1, **caractérisé par le fait que** le dispositif d'entraînement ESM (10) comprend une commande maître (11; 11') qui, en réponse à une constatation d'un écart entre la valeur réelle et la valeur de consigne dans l'une au moins des branches de ligne (4), commande ou règle la valeur réelle y présente à la valeur de consigne y présente.

3. Dispositif d'entraînement ESM (10) selon la revendication 1 ou 2, **caractérisé par le fait que** le dispositif d'entraînement ESM (10) comprend une mémoire dans laquelle est mémorisée la valeur de consigne pour ladite au moins une branche de ligne (4).

4. Dispositif d'entraînement ESM (10) selon la revendication 1, 2 ou 3, **caractérisé par le fait que** la saisie de valeur réelle se fait en soustrayant la valeur réelle saisie dans la branche de ligne (4) respective de la valeur réelle saisie dans une autre branche de ligne (4) ou d'une valeur moyenne des valeurs réelles saisies dans plusieurs des branches de ligne (4), en particulier en soustrayant la valeur réelle dans la branche de ligne (4) respective de la valeur réelle dans la branche de ligne (4) qui s'étend de ladite unité de génération de stimuli ESM (3) à l'électrode ESM (1) laquelle forme conjointement avec l'électrode ESM (1) disposée dans la branche de ligne (4) à compenser une paire d'électrodes ESM (1) associées l'une à l'autre dans un circuit à travers le corps vivant, dans lequel ledit dispositif de comparaison (13) fixe de préférence à zéro la valeur de consigne pour ladite au moins une branche de ligne (4) à compenser.

5. Dispositif d'entraînement ESM (10) selon l'une quelconque des revendications précédentes, **caractérisé par le fait que** la saisie de valeur réelle se fait par mesure d'une différence de potentiel entre deux branches de ligne (4) ayant des électrodes ESM appariées, dans de tels segments de temps des intervalles entre impulsions entre les stimuli ESM dans le schéma d'excitation, dans lesquels aucune alimentation en courant de compensation n'a lieu dans les deux branches de ligne (4).

6. Dispositif d'entraînement ESM (10) selon l'une quelconque des revendications précédentes, **caractérisé par le fait que** ledit dispositif de compensation (14) pour ladite au moins une branche de ligne (4) comprend un dispositif d'augmentation de tension et/ou d'intensité de courant par le biais duquel la tension et/ou l'intensité de courant dans la branche de ligne (4) est augmentée dans le cas d'un écart entre la valeur réelle et la valeur de consigne, en réponse à un input d'utilisateur ou par la commande (11).

7. Dispositif d'entraînement ESM (10) selon la revendication 6, **caractérisé par le fait que** la compensation se fait par une augmentation des amplitudes de l'intensité de courant et/ou de la tension des stimuli ESM.

8. Dispositif d'entraînement ESM (10) selon la revendication 6 ou 7, **caractérisé par le fait que** la compensation se fait dans de tels segments de temps des intervalles entre impulsions entre les stimuli ESM dans le schéma d'excitation, dans lesquels aucune mesure n'est effectuée dans la branche de ligne (4) respective à compenser, dans lequel la compensation se fait par un courant cadencé qui est amené à la branche de ligne (4) respective à compenser et qui est inférieur par rapport aux amplitudes de l'intensité de courant des stimuli ESM.

9. Dispositif d'entraînement ESM (10) selon l'une quelconque des revendications précédentes, **caractérisé par le fait que** ledit dispositif de compensation (14) pour ladite au moins une branche de ligne (4) comprend une électrode de protection (6) qui est montée sur ou à proximité de l'électrode ESM (1) de cette branche de ligne (4) et reliée via une ligne séparée (15) à une source de courant et qui, dans le cas d'un écart entre la valeur réelle et la valeur de consigne, en réponse à un input d'utilisateur ou par la commande / asservissement (11'), est alimentée en courant.

10. Dispositif d'entraînement ESM (10) selon la revendication 9, **caractérisé par le fait que** la saisie de valeur réelle se fait par mesure d'une différence de potentiel entre la branche de ligne (4) respective à compenser et l'électrode de protection (6) associée à son électrode ESM (1), dans de tels segments de temps des intervalles entre impulsions entre des stimuli ESM dans le schéma d'excitation, dans lesquels aucune alimentation en courant de compensation n'a lieu dans la branche de ligne (4) respective.

11. Dispositif d'entraînement ESM (10) selon la revendication 9 ou 10, **caractérisé par le fait que** la compensation se fait par un asservissement de la tension et/ou de l'intensité de courant dans la branche de ligne (4) respective lorsqu'il y a un écart entre la différence de potentiel et une différence de potentiel souhaitée entre cette branche de ligne (4) et l'électrode de protection (6) associée.

12. Dispositif d'entraînement ESM (10) selon l'une quelconque des revendications 9 à 11, **caractérisé par le fait que** l'électrode de protection (6) est réalisée à partir d'un métal noble, d'un alliage de métal noble, de titane revêtu de métal noble ou d'oxydes mixtes ou d'un alliage de titane revêtu de métal noble ou d'oxydes mixtes, dans lequel, dans un agencement avantageux de l'électrode de protection (6) directement sur l'électrode ESM (1) associée, une couche isolante est présente entre ladite électrode de protection (6) et l'électrode ESM (1).

13. Dispositif d'entraînement ESM (10) selon l'une quelconque des revendications précédentes, **caractérisé par le fait que**, dans chacune des branches de ligne (4), l'écart entre la valeur réelle et la valeur de consigne est compensé sur la base d'une résistance mesurée ou déterminée ou bien d'une grandeur mesurée ou déterminée qui correspond à la résistance, en réponse à un input d'utilisateur ou par la commande (11; 11'), dans lequel, avantageusement, au moins un propre dispositif de mesure (12), de préférence aussi un propre dispositif de compensation (14; 6, 15) et/ou un dispositif de comparaison (13) est prévu pour chaque branche de ligne (4).

14. Dispositif d'entraînement ESM (10) selon l'une quelconque des revendications précédentes, **caractérisé par le fait que** les électrodes ESM (1) présentent chacune un pad plat s'appliquant de manière flexible contre le corps ou une portion de structure textile ayant une couche conductrice électroconductrice, en particulier une couche conductrice contenant des fils métalliques ou métallisés, que les électrodes ESM (1) portent chacune une électrode de protection (6) qui est reliée ou peut être reliée de manière électroconductrice à ladite unité de génération de stimuli ESM (3).

15. Procédé de protection d'un dispositif d'entraînement ESM (10) contre la corrosion - provoquée par le fonctionnement du dispositif d'entraînement ESM (10), en particulier d'un dispositif d'entraînement ESM (10) selon l'une quelconque des revendications précédentes - d'une électrode ESM (1) et/ou d'autres éléments (5) sensibles à la corrosion, tels qu'un bouton-poussoir, dans au moins une branche de ligne (4), dans lequel, durant le fonctionnement du dispositif d'entraînement ESM (10), les électrodes ESM (1) de celui-ci qui peuvent être appliquées au corps vivant sont reliées chacune de manière électroconductrice à son unité de génération de stimuli ESM (3) afin de former des branches de ligne (4) qui, pendant l'entraînement ESM, sont complétées à travers le corps de manière à avoir un ou plusieurs circuits fermés, en particulier respectivement une paire des branches de ligne (4) étant associées l'une à l'autre afin de former, durant l'entraînement ESM, un circuit fermé s'étendant à travers le corps, **caractérisé par le fait qu'**une résistance ou une grandeur correspondant à la résistance est mesurée en tant que valeur réelle, ladite valeur réelle dans la branche de ligne (4) est comparée à une valeur de consigne mise en mémoire ou déterminée, et, lorsqu'il y a un écart entre la valeur réelle et la valeur de consigne, l'écart est compensé, dans lequel on procède en particulier selon les caractéristiques de l'une des revendications 4, 5, 7, 8, 11, 12.
